# EUROPEAN PATENT SPECIFICATION

(11) **EP 1 764 051 B1**
(45) Date of publication and mention of the grant of the patent: **08.08.2012**
(21) Application number: 06120836.9
(22) Date of filing: 18.09.2006
(51) Int. Cl.: A61B 17/80, A61B 17/70

(54) **Plate structure for fracture fixation**
Plattenstruktur für Frakturfixierung
Structure de plaque pour fixation de fracture

(30) Priority: 19.09.2005 TR 200503744
(43) Date of publication of application: 21.03.2007
(73) Proprietor: HAVITCIOGLU, Hasan, Izmir (TR)
(72) Inventor: HAVITCIOGLU, Hasan, Izmir (TR)
(74) Representative: Iskender, Ibrahim

(56) References cited:
- US-A- 3 547 114
- US-A1- 2003 229 348
- US-A1- 2004 073 215
- US-A1- 2005 085 814

## Description

### Technical Field

The invention relates to the metal plates employed for the fixation of the fractures that could occur in human body.

The invention relates in particular to the rearrangement and physiological application of the plates used in the fracture fixation.

### Background of the Invention

Various plate structures have been realized for the fixation of the fractures in the human body. The fractures are encountered in the human body for various reasons. Metal plates are frequently used for the treatment of said fractures. When its historical development is examined, the plates have widely been used in the fracture fixation since the beginning of this century. The plates used in the fracture fixation usually result in recovery. However, the use of the metal plates leads to some problems and recovery becomes delayed or is never achieved, due to numerous reasons in some cases.

Since 1970's, 316 L had widely been used as the steel material, then various materials were tried for application, such as titanium and the alloys thereof. However, as in the steel material, some problems were encountered also in the case of titanium alloys. The efforts have been made to develop the plates with such material and shape to fit the load to be imparted on said plate at the place where they will be used in the human body. For example, the plates for use in lower extremity is preferred to have a thick and strong material, while a thinner material resistant to less load is preferred for the upper extremity.

Although 316 L steel material has its advantages since it can bear many various loads imparted on the bone until the knitting of the fracture has been achieved, owing to its elasticity module being about 200 GPa, based on the knowledge that the elasticity module is about 20 GPa for the bone cortex, there is a phenomenon we call stress shielding leading to the weakening of the bone, where 316 L steel bears many loads exerted on the body.

Different materials have been used. Regarding the form, there are many plate designs available.

There also exist many studies where efforts have been shown to alter the surface properties of the material, in a view to increase its bio-compatibility and the durability.

Despite the use of many forms and different materials, for which there are efforts to improve, 316 L is still the most preferred material owing to such factors as its ease of technical processing and its cheap price.

The prior art which is considered to be relevant is mentioned as follows:
US 2003229348**:** A connecting device for use in attaching together at least two adjacent vertebrae, comprising first and second plate members adapted to be fixedly secured respectively to upper and lower vertebrae of a pair of adjacent vertebrae, said first and second plate members being provided with respective first and second mating members, said first and second mating members being engaged so as to allow a relative displacement between said first and second plate members in response to a similar relative displacement between the upper and lower vertebrate that alters a spacing therebetween.
US 2004073215**:** An osteosynthesis device for the vertebral column, the device comprising at least one elastically deformable connection system connected at opposite ends to at least first and second fixing portions suitable for being fixed to vertebrae by means of bone anchoring elements, wherein the elastically deformable connection system comprises: a deformable connection member presenting: in a "sagittal" plane, determined stiffness for exerting a return force on flexion-extension movements between the fixing portions; in a "frontal" plane perpendicular to the sagittal plane, determined stiffness for exerting a return force on lateral inflexion movements between the fixing portions, the stiffness of the deformable connection member in the frontal plane being less than its stiffness in the sagittal plane; and along an axis defined by the intersection between the sagittal and frontal planes determined stiffness for exerting a return force on traction-compression movements between the fixing portions; and means for limiting flexion-extension, traction-compression, and lateral inflexion movements between the fixing portions.
US 2005085814**:** An orthopedic device for securing two or more bone portions, said device comprising: an elongate member configured for engagement to the two or more bone portions and allowing translational or rotational movement for a first one of the two or more bone portions relative to a second one of the two or more bone portions; a reinforcing component composed of a biodegradable material and engaged to the elongate member to inhibit the translational or rotational movement for a first one of the two or more bone portions relative to a second one of the two or more bone portions; and at least one bone fastener for fixedly securing the elongate member to at least one of the two or more bone portions.

### The Object of the Invention

Based on the known state of the art, the object of the invention is to eliminate the present disadvantages owing to the improvements provided in the plates used for the fixation of the fractures that may occur in the human body.

Another object of the invention is to provide a higher degree of physiological use for the plates employed in the human body, and to provide better load distributions between the plate and the bone.

Another object of the invention is to enable the plates to stay in the human body for a longer time and to reduce the need for a new surgical intervention. Another object of the invention is to provide better physiological knitting by ensuring flexibility in the use of the plate being fixed, without any loss in the durability and the strength of the material.

Another object of the invention is to provide a use for the plate fixed whereby the bone weakening (bone shielding) is prevented without any loss in durability and strength of the material, while the flexibility of use is achieved. Another object of the invention is to provide a planning and development to be implemented under conditions more suitable for human body, by adding elasticity to the system without loosing the flexibility of the plate and sacrificing the strength of the material used.

In order to achieve the said objects, innovations have been provided in the plates having at least one fixation body employed for the fixation of the fractures that may occur in the human body.

According to a preferred embodiment of the invention, at least one lower body has been provided, which is connected with said fixation body at the lower end, in order to provide flexibility to said fixation body, to provide physiological knitting and to keep the bone deformation at a minimum level.

According to a preferred embodiment of the invention, at least one suspension element capable of shock absorbing is provided between said fixation body and the lower body, in order to provide flexibility to said fixation body, to provide physiological knitting and to keep the bone deformation at a minimum level. According to a preferred embodiment of the invention, said shock absorbing element is viscoelastic material. In this way, the plates to be employed in the human body are enabled for use as a more flexible structure, despite their elasticity module remaining constant. Consequently, a more balanced load is provided to be exerted on the bone.

According to a preferred embodiment of the invention, at least one housing formed on said lower body, at least one projecting part formed on the fixation body, which is the other part of the lower body, and corresponding to said housing and at least one shock absorbing element located between said housing and the projecting part are provided to form said suspension element.

According to a preferred embodiment of the invention, at least one thrust part is provided in the form of a projection on the side of the lower body facing the fixation body, in order to limit the movement of said fixation body and lower body during stretching and to prevent the suspension element from coming out of the housing.

### Description of the Drawings

Figure-1 is the front sectional view of the plate according to an illustrative embodiment of the invention.
Figure-2 is the view of the different application of the plate to the fracture line depending on the hole location and quantity, according to an illustrative embodiment of the invention.

### Reference Numbers

| | | | |
|---|---|---|---|
| 1 | Fixation body | 2.2 | Thrust projection |
| 1.1 | Projecting part | 2.3 | Thrust guide |
| 2 | Lower body | 3 | Shock absorbing element |
| 2.1 | Housing | 4 | Plate pin hole |

### Detailed Description of the Invention

The embodiment of the invention provided in the figures is the metal plate applied for the fixation of the fractures that may happen in the human body as a result of accidents, injuries, physiological reasons and similar conditions. The plate fixation body (1) and the upper body (2) seen in Figure-1 are connected with the bone by means of the plate pin holes (4). The flexibility is provided for the fixation body (1) by means of a lower body (2) connected to the fixation body (1) at the lower end. By means of the body having two parts, physiological knitting is ensured and the bone deformation is kept to a minimum.
Owing to a suspension element (3) capable of shock damping between the fixation body (1) and lower body (2), the flexibility is provided for the fixation body (1), a physiological knitting is achieved and the bone deformation is kept to a minimum. In this way, the possibility is obtained for the plates to be used in the human body in a flexible structure, while their elasticity module remains constant. As a result, a more balanced load is provided to be exerted on the bone.

The thrust projection (2.2) (upper end) of the lower body (2) has a suitable form such that it will correspond to the lower end of the plate body (1). A projecting part (1.1) is formed to correspond to the housing (2.1) made on the lower body (2), which is the other part of the fixation body (1). The housing (2.1) formed on the lower body (2) has a form to accommodate the shock absorbing element (3) and the projecting part (1.1). The desired flexibility is provided by means of a shock absorbing element (3) located between the housing (2.1) and the projecting part (1.1).

After the projecting end (1.1) of the plate fixation body (1) has contacted with the shock absorbing element (3), the micro movement is provided to the system owing to the elasticity of the shock absorbing element (3) under the load, and this enhances the fracture knitting. This property is a new feature due to the system's gaining flexibility, in addition to the durability of the plate. The damping flexible materials like viscoelastic material is used as the shock absorbing element (3) placed in said housing (2.1), in order to provide micro movement to the plate bodies (1, 2).

On the side of the lower body (2) facing the fixation body (1), the movement of the fixation body (1) and the lower body (2) is limited during the stretching and the suspension element (3) is prevented from coming out of the housing (2.1), owing to a thrust part (2.2) in the form of a projection. The thrust guide (2.3) also provides guidance for the elastic movement of the fixation body (1). When the thrust guide (2.3) and the thrust projection (2.2) and the corresponding surfaces on the fixation body (1) come in contact, there will have left no damping effect of the shock absorbing element (3) between the fixation body (1) and the lower body (2) and there will take place the direct force transfer.
In Figure-2, the application of elasticity to the plate at different locations and with different quantities is shown according to a different embodiment of the plate. The elasticity may be provided to the plate and the system at location different than the fracture site, without sacrificing the stability of the whole system (the fracture ends and the plate). As seen in Figure-2, the size of the fixation body (1) and the lower body (2) may be changed when necessary. The location and the quantity of the plate pin holes (4) used for securing may also be changed accordingly.

The invention may not be limited to the illustrative embodiments provided in this section. The alternative embodiments that may be performed by the persons skilled in the art based on the basic principles within the protective scope as defined in the claims will mean the violation of the invention.

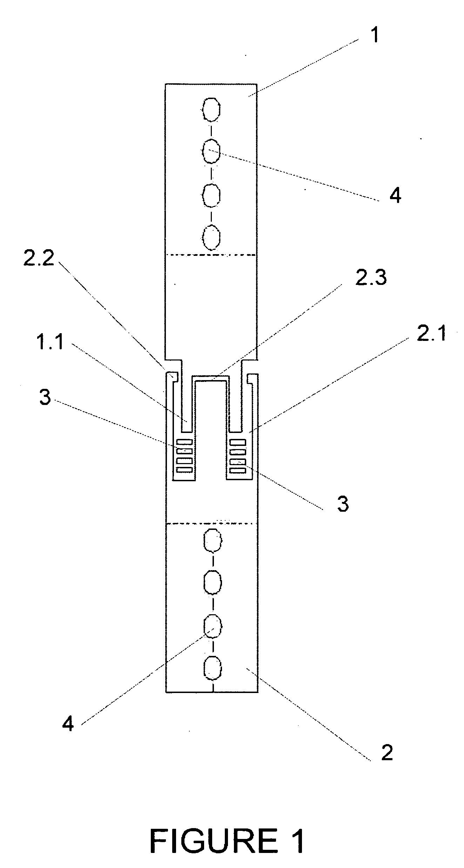

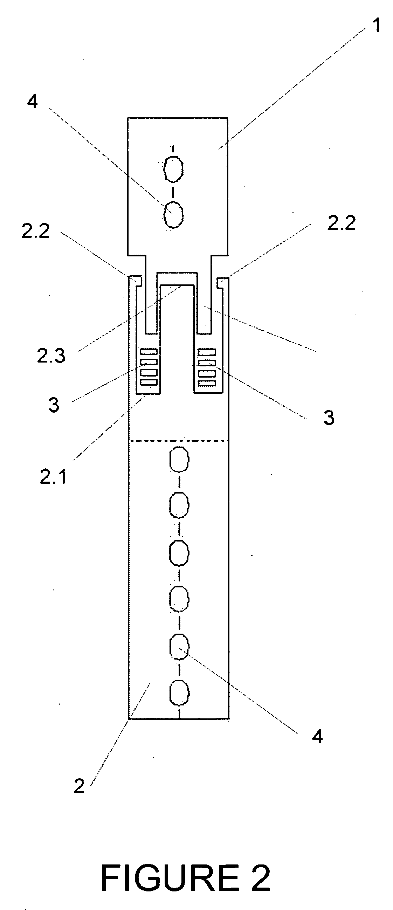

## Claims

1. A plate having at least one fixation body (1) applied for the fixation of the fractures that may occur in the human body and at least one lower body (2) connected with said fixation body (1) at the lower end, in order to provide flexibility for said fixation body (1), to achieve a physiological knitting and to keep the bone deformation to a minimum
- at least one housing (2.1) formed on said lower body (2),
- at least one projecting part (1.1) formed on the fixation body (1), which is the other part of the lower body (2), and corresponding to said housing (2.1),
- at least one suspension or shock absorbing element (3) capable of shock damping between said fixation body (1) and the lower body (2),
**characterized in that** the element (3) is viscoelastic material and located between said housing (2.1) and the projecting part (1.1) and in said housing (2.1).

2. A plate according to claim 1, **characterized in that** said lower body (2) comprises on its side facing the fixation body (1) at least one thrust part (2.2) in the form of a projection.

## Patentansprüche

1. Platte, die mindestens einen Fixationskörper (1), der für die Fixierung von den Frakturen, die in dem menschlichen Körper auftreten können, eingesetzt wird, und mindestens ein Körperunterteil (2) aufweist, welches mit dem Fixationskörper (1) an dem unteren Ende verbunden ist, um eine Beweglichkeit für den Fixationskörper (1) bereitzustellen, damit eine physiologische Zusammenfügung erreicht wird und die Knochenverformung auf ein Mindestmaß beschränkt bleibt;
- mindestens eine Einhausung (2.1) aufweist, die an dem Körperunterteil (2) ausgebildet ist,
- mindestens ein vorspringendes Teilstück (1.1) aufweist, das an dem Fixationskörper (1) ausgebildet ist, welches das andere Teil zu dem Körperunterteil (2) und das Gegenstück zu der Einhausung (2.1) ist,
- mindestens ein Federungselement oder ein Dämpfungselement (3) aufweist, welches zur Stoßdämpfung zwischen dem Fixationskörper (1) und dem Körperunterteil (2) in der Lage ist,
**dadurch gekennzeichnet, dass** das Element (3) ein viskoelastisches Material ist und zwischen der Einhausung (2.1) und dem Druckfortsatz (1.1) und in der Einhausung (2.1) ausgebracht ist.

2. Platte nach Anspruch 1, **dadurch gekennzeichnet, dass** das Körperunterteil (2) an seiner Seite, die dem Fixationskörper (1) zugewandt ist, mindestens ein Druckteil (2.2) in der Form eines Fortsatzes aufweist.

## Revendications

1. Plaque comportant au moins un corps de fixation (1) appliqué pour la fixation des fractures pouvant survenir dans le corps humain et au moins un corps inférieur (2) connecté audit corps de fixation (1) à son extrémité inférieure afin de procurer de la souplesse audit corps de fixation (1) afin de réaliser un tricotage physiologique et de maintenir la déformation osseuse à un minimum ;
- au moins un logement (2, 1) formé sur ledit corps inférieur (2),
- au moins une partie saillante (1.1) formée sur le corps de fixation (1), qui est l'autre partie du corps inférieur (2), et correspondant audit logement (2.1)
- au moins un élément de suspension ou d'absorption de chocs (3) capable d'amortir les chocs entre ledit corps de fixation (1) et le corps inférieur (2),
**caractérisé en ce que** l'élément (3) est un matériau viscoélastique et se situe entre ledit logement (2.1) et la partie saillante (1.1) et dans ledit logement (2.1).

2. Plaque selon la revendication 1, **caractérisée en ce que** ledit corps inférieur (2) comporte sur son côté faisant face au corps de fixation (1) au moins une partie axiale (2.2) ayant la forme d'une saillie.
